(19) Europäisches Patentamt
European Patent Office
Office européen des brevets

(11) Veröffentlichungsnummer: **0 000 346**
**B1**

(12) # EUROPÄISCHE PATENTSCHRIFT

(21) Anmeldenummer: **78100253.0**

(22) Anmeldetag: **28.06.78**

(51) Int. Cl.³: **C 07 D 241/42,** C 07 D 241/44, C 07 D 401/10, C 07 D 403/10, C 07 D 405/10, C 07 D 409/10, C 07 D 413/10, C 07 D 417/10, C 08 K 5/29, D 06 L 3/12

(54) **Chinoxalinverbindungen, Verfahren zu deren Herstellung, deren Verwendung zum Weisstönen organischer Materialien und damit weissgetönte Materialien.**

(30) Priorität: **07.07.77 DE 2730644**

(43) Veröffentlichungstag der Anmeldung:
**24.01.79 Patentblatt 79/02**

(45) Bekanntmachung des Hinweises auf die Patenterteilung:
**09.01.80 Patentblatt 80/01**

(84) Benannte Vertragsstaaten: **CH DE FR GB**

(56) Entgegenhaltungen:
**FR – A – 2 206 951**

(73) Patentinhaber: **BAYER Aktiengesellschaft,**
**Zentralbereich Patente, Marken und Lizenzen**
**Bayerwerk,**
**D–5090 Leverkusen 1 (DE)**

(72) Erfinder: **Eckstein, Udo, Dr.,**
**Morgengraben 2**
**D–5000 Köln 80 (DE)**
Erfinder: **Theidel, Hans, Dr.,**
**Tempelhofer Strasse 66,**
**D–5090 Leverkusen 1 (DE)**

**Anmerkung:** Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 346 B1

# Chinoxalverbindungen, Verfahren zu deren Herstellung, deren Verwendung zum Weisstönen organischer Materialien und damit weissgetönte Materialien

Gegenstand der Erfindung sind Fluoreszenz-Farbstoffe, Verfahren zu ihrer Herstellung und ihre Verwendung zum Weißtönen organischer Materialien.

Die neuen Verbindungen entsprechen der Formel

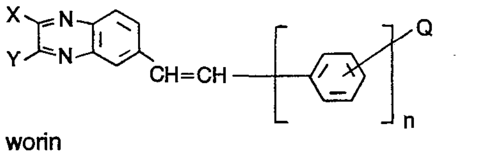

(I)

worin

X und Y Wasserstoff, Halogen, Alkyl, Aralkyl, Alkenyl, Hydroxy, Amino, Alkoxy, Aralkoxy, Cycloalkoxy, Aryloxy, Alkylmercapto, Alkylamino, Dialkylamino, Morpholino, Piperidino, Piperazino, Pyrrolidino, Acylamino oder Arylamino,

Q Wasserstoff, Pyrazol-1-yl, Oxazol-2-yl, Benzoxazol-2-yl, Naphthoxazol-2-yl, 1,2,4-Oxadiazol-5-yl, 1,3,4-Oxadiazol-2-yl, Isoxazol-3-yl, Isoxazol-5-yl, Thiazol-2-yl, Benzthiazol-2-yl, 1,3,4-Thiadiazol-2-yl, Imidazol-2-yl, Benzimidazol-2-yl, 1,2,3-Triazol-2-yl, 1,2,3-Triazol-4-yl, 1,2,4-Triazol-3-yl, 1,2,4-Triazol-5-yl, 1,3,5-Triazin-2-yl, 2H-Benzotriazol-2-yl, 2H-Naphthotriazol-2-yl, 1,2,3,4-Tetrazol-5-yl, 1,2,3,4-Tetrazol-1-yl, Benzo[b]-furan-2-yl, Naphtho[2,1-b]-furan-2-yl, Benzo[b]-thiophen-2-yl, Naphtho[2,1-b]-thiophen-2-yl, Pyrimidin-2-yl, Pyridin-2-yl, Chinazolin-4-yl oder Chinazolin-2-yl und

n 1 oder 2 bedeuten, wobei die Substituenten X, Y, Q und die übrigen cyclischen Reste durch für Weißtöner übliche nicht-chromophore Substituenten weiter substituiert sein können. Vorzugsweise ist n 1.

Nicht-chromophore Substituenten sind beispielsweise Halogen, gegebenenfalls substituiertes Alkyl, gegebenenfalls substituiertes Alkenyl, Aryl, Aralkyl, gegebenenfalls substituiertes Alkoxy, Alkoxycarbonyl, gegebenenfalls substituiertes Aminocarbonyl, Cyan, Alkylsulfonyl, Alkoxysulfonyl, gegebenenfalls substituiertes Aminosulfonyl, Acyl, Acylamino, Hydroxy, Aryloxy, Aralkyloxy, Alkenyloxy, Aryloxycarbonyl, Aralkyloxycarbonyl, Carboxy oder Acyloxy.

Alkyl ist insbesondere $C_1$–$C_4$-Alkyl, das durch Hydroxy, $C_1$–$C_4$-Alkoxy, Cyan, Carboxy, $C_1$–$C_4$-Alkoxycarbonyl, Aminocarbonyl, Chlor oder Brom monosubstituiert sein kann oder Trifluormethyl.

Alkenyl ist insbesondere $C_2$–$C_5$-Alkenyl, das durch Hydroxy, $C_1$–$C_4$-Alkoxy, Cyan, Carboxy, $C_1$–$C_4$-Alkoxycarbonyl, Chlor oder Brom monosubstituiert sein kann.

Halogen ist insbesondere Fluor, Chlor und Brom, vorzugsweise Chlor. Aryl ist insbesondere gegebenenfalls durch $C_1$–$C_4$-Alkyl, Trifluormethyl, Chlor, Brom Carboxy, Cyan, $C_1$–$C_4$-Alkoxycarbonyl oder $C_1$–$C_4$-Alkoxy substituiertes Phenyl.

Aralkyl ist insbesondere Phenyl-$C_1$–$C_4$-Alkyl, das im Phenylkern noch durch Chlor, Methyl oder Methoxy substituiert sein kann.

Alkoxy ist insbesondere $C_1$–$C_4$-Alkoxy oder ein Rest der Formel

$$-(OCH_2-CH_2)_m-OR$$

wobei

R Wasserstoff oder $C_1$–$C_4$-Alkyl und

m eine ganze Zahl von 1 bis 20 bedeutet.

Cycloalkyloxy ist insbesondere Cyclopentyloxy und Cyclohexyloxy.

Acyl ist insbesondere $C_1$–$C_4$-Alkylcarbonyl, $C_1$–$C_4$-Alkylsulfonyl, gegebenenfalls durch Methyl, Methoxy oder Chlor substituiertes Benzoyl oder gegebenenfalls durch Methyl, Methoxy oder Chlor substituiertes Benzolsulfonyl.

Als Substituenten der Aminocarbonyl- und Aminosulfonylreste kommen insbesondere $C_1$–$C_4$-Alkyl, gegebenenfalls durch Methyl, Methoxy oder Chlor substituiertes Phenyl oder Phenyl-$C_1$–$C_4$-alkyl in Frage.

Bevorzugte Verbindungen entsprechen der Formel

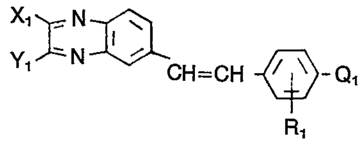

(II)

worin

$X_1$ und $Y_1$ Wasserstoff, Chlor, $C_1$–$C_4$-Alkylamino, Di-$C_1$–$C_4$-Alkylamino, Morpholino, Piperidino, gegebenenfalls durch Methyl, Methoxy oder Chlor substituiertes Phenylamino oder einen Rest der Formel

$$-(OCH_2-CH_2)_q-OR_2$$

$R_1$ Wasserstoff, Chlor, $C_1$–$C_4$-Alkyl, $C_1$–$C_4$-Alkoxy, $C_1$–$C_4$-Alkoxycarbonyl oder Cyano,

$R_2$ Wasserstoff, $C_1$–$C_4$-Alkyl, Benzyl oder Phenyl,

q eine ganze Zahl von 0 bis 7 und

$Q_1$ Chlor, Brom, Cyano, Carboxy, $C_1$–$C_4$-Alkoxycarbonyl oder einen Rest der Formel

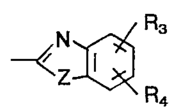 , 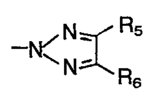 ,

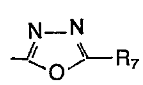 , 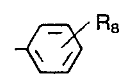

$R_3$ Wasserstoff, Chlor, $C_1$–$C_4$-Alkyl, Phenyl-$C_1$–$C_3$-alkyl, Cyclohexyl, Phenyl, $C_1$–$C_4$-Alkoxy, $C_1$–$C_4$-Alkylsulfonyl, $C_1$–$C_4$-Alkoxycarbonyl, Cyan oder Carboxy oder zusammen mit $R_4$ einen gegebenenfalls durch 1 bis 4 Methylgruppen substituierten ankondensierten 1-Cyclopenteno-, 1-Cyclohexeno- oder Benzoring,

$R_4$ Wasserstoff, Chlor oder Methyl oder zusammen mit $R_3$ einen gegebenenfalls durch 1 bis 4 Methylgruppen substituierten ankondensierten 1-Cyclopenteno-, 1-Cyclohexeno- oder Benzoring,

$R_5$ $C_1$–$C_4$-Alkyl, Phenyl oder Styryl oder zusammen mit $R_6$ einen gegebenenfalls durch $C_1$–$C_4$-Alkyl, $C_1$–$C_4$-Alkoxy oder Chlor substituierten ankondensierten Benzoring oder ankondensiertes Naphtho,

$R_6$ Wasserstoff, $C_1$–$C_4$-Alkyl oder Phenyl oder zusammen mit $R_5$ einen gegebenenfalls durch $C_1$–$C_4$-Alkyl, $C_1$–$C_4$-Alkoxy oder Chlor substituierten ankondensierten Benzoring oder ankondensiertes Naphtho,

$R_7$ gegebenenfalls durch $C_1$–$C_4$-Alkyl, $C_1$–$C_4$-Alkoxy, $C_1$–$C_4$-Alkoxycarbonyl, Cyan oder Chlor substituiertes Phenyl, Styryl, Biphenylyl oder Naphthyl,

$R_8$ Wasserstoff, $C_1$–$C_4$-Alkyl, $C_1$–$C_4$-Alkoxy, $C_1$–$C_4$-Alkoxycarbonyl, Cyan oder einen gegebenenfalls durch $C_1$–$C_4$-Alkoxycarbonyl, Cyan oder Chlor substituierten Benzoxazol-2-yl-Rest,

Z O, S oder $NR_9$ und

$R_9$ Wasserstoff, $C_1$–$C_4$-Alkyl, Acetyl, Benzoyl, Benzyl oder Phenyl bedeuten.

Besonders wertvolle Verbindungen entsprechen der Formel

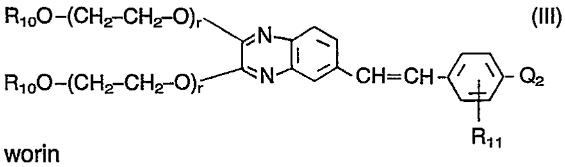

(III)

worin

$R_{10}$ $C_1$–$C_4$-Alkyl,

$R_{11}$ Wasserstoff oder Cyan,

r eine ganze Zahl von 0 bis 2,

$Q_2$ einen Rest der Formeln

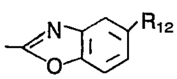 , 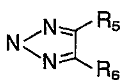 , 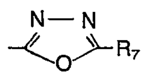

$R_{12}$ Wasserstoff, Chlor, $C_1$–$C_4$-Alkyl, Phenyl-$C_1$–$C_3$-alkyl, Cyclohexyl, Phenyl, $C_1$–$C_4$-Alkoxy, $C_1$–$C_4$-Alkylsulfonyl, $C_1$–$C_4$-Alkoxycarbonyl, Cyan oder Carboxy bedeuten und

$R_5$, $R_6$ und $R_7$ die vorstehend genannte Bedeutung haben.

Die erfindungsgemäßen Fluoreszenzfarbstoffe lassen sich auf verschiedenen Wegen herstellen. Vorzugsweise kondensiert man

a) eine Phosphono-Verbindung der Formel

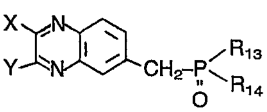

(IV)

worin

X und Y die vorstehend genannte Bedeutung haben und der Benzolring weitere nicht-chromophore Substituenten aufweisen kann,

$R_{13}$ und $R_{14}$ $C_1$–$C_4$-Alkoxy, $C_5$–$C_6$-Cycloalkoxy, Phenoxy oder Phenyl bedeuten, mit einem Aldehyd der Formel

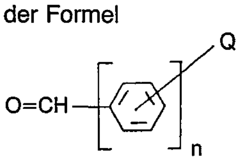

(V)

worin

Q und n die vorstehend genannte Bedeutung besitzen und der Benzolring weitere nicht-chromophore Substituenten aufweisen kann, oder

b) eine Phosphono-Verbindung der Formel

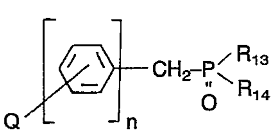

(VI)

worin

Q, $R_{13}$, $R_{14}$ und n die vorstehend genannte Bedeutung besitzen und der Benzolring durch nicht-chromophore Substituenten substituiert sein kann, mit einem Aldehyd der Formel

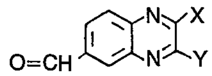

(VII)

worin

X und Y die vorstehend genannte Bedeutung haben und der Benzolring durch nicht-chromophore Substituenten substituiert sein kann, in organischen Lösungsmitteln in Gegenwart basischer Kondensationsmittel.

Als Lösungsmittel wählt man vorteilhaft indifferente Lösungsmittel, beispielsweise Kohlenwasserstoffe wie Toluol oder Xylol oder Alkohole wie Methanol, Äthanol, Isopropanol, Butanol, Glykol, Glykoläther wie 2-Methoxyäthanol; Hexanol, Cyclohexanol, Cyclooctanol, ferner Äther wie Diisopropyläther, Dioxan, Tetrahydrofuran, weiterhin Formamide oder N-Methylpyrrolidon. Besonders geeignet sind dipolare organische Lösungsmittel wie Dimethylformamid und Dimethylsulfoxid.

Als Kondensationsmittel kommen stark basische Verbindungen in Betracht wie Alkali- oder Erdalkalimetallhydroxide, Alkali- oder Erdalkaliamide und Alkali- oder Erdalkalimetallalkoholate, beispielsweise Kaliumhydroxid, Natriumhydroxid, Kalium-tert.-butylat, Natriumamid oder Natriummethylat, ferner die Alkaliverbindungen des Dimethylsulfoxids und Alkalihydride sowie gegebenenfalls Alkalimetalldispersionen. Man arbeitet bevorzugt im Temperaturbereich von 0 bis 100° C.

Die erfindungsgemäßen Verbindungen werden ebenfalls erhalten, wenn man

c) anstelle der Phosphonoverbindungen (IV) und (VI) die entsprechenden quartären Phosphonium-Salze, beispielsweise die Triphenylphosphonium-Salze, einsetzt und diese nach Wittig über die Stufe der Phosphorylene mit den Aldehyden (V) und (VII) kondensiert oder

d) die entsprechenden Aldehydanile in Dimethylformamid nach Siegrist in Gegenwart von basischen Kondensationsmitteln mit den 6-Methylchinoxalinen umsetzt.

An den Reaktionsprodukten der vorstehenden Verfahren können noch weitere an sich bekannte Umwandlungen vorgenommen werden wie Halogenierungen, funktionelle Abwandlungen von Carboxylgruppen, Einführung von Chlormethylgruppen oder Austausch von Halogenatomen gegen Cyanogruppen.

Die erfindungsgemäßen Verbindungen sind aufgrund ihrer Absorption im ultravioletten Bereich und ihrer Fluoreszenz geeignet zum Weißtönen der verschiedensten synthetischen, halbsynthetischen und natürlichen organischen hochmolekularen Materialien, wie sie im folgenden einzeln angegeben sind.

I. Synthetische organische hochmolekulare Materialien:

a) Polymerisationsprodukte auf Basis mindestens eine polymerisierbare Kohlenstoff-Kohlenstoff-Doppelbindung enthaltender organischer Verbindungen, d. h. deren Homo- oder Copolymerisate sowie deren Nachbehandlungsprodukte wie beispielsweise Vernetzungs-, Pfropfungs- oder Abbauprodukte, Polymerisat-Verschnitte, usw., wofür beispielsweise genannt seien: Polymerisate auf Basis von $\alpha,\beta$-ungesättigten Carbonsäuren, insbesondere von Acrylverbindungen (wie z. B. Acrylestern, Acrylsäuren, Acrylnitril, Acrylamiden und deren Derivaten oder deren Methacryl-Analoga), von Olefin-Kohlenwasserstoffen (wie z. B. Aethylen, Propylen, Isobutylen, Styrole, Diene wie besonders Butadien, Isopren, d. h. also auch Kautschuke und kautschukähnliche Polymerisate, ferner sogenannte ABS-Polymerisate), Polymerisate auf Basis von Vinyl- und Vinyliden-Verbindungen (wie z. B. Vinylestern, Vinylchlorid, Vinylsulfonsäure, Vinyläther, Vinylalkohol, Vinylidenchlorid, Vinylcarbazol), von halogenierten Kohlenwasserstoffen (Chloropren, nachhalogenierte Aethylene), von ungesättigten Aldehyden und Ketonen (z. B. Acrolein usw.), von Allylverbindungen usw., Pfropfpolymerisationsprodukte (z. B. durch Aufpfropfen von Vinylmonomeren), Vernetzungsprodukte (beispielsweise mittels bi- oder mehrfunktionellen Vernetzern wie Divinylbenzol, mehrfunktionelle Allylverbindungen oder Bisacrylverbindungen) oder durch partiellen Abbau (Hydrolyse, Depolymerisation) oder Modifizierung reaktiver Gruppierungen (z. B. Veresterung, Verätherung, Halogenierung, Selbstvernetzung) erhältlich sind.

b) Andere Polymerisationsprodukte wie z. B. durch Ringöffnung erhältlich, z. B. Polyamide vom Polycaprolactam-Typ, ferner Formaldehyd-Polymerisate oder Polymere, die sowohl über Polyaddition als auch Polykondensation erhältlich sind, wie Polyäther, Polythioäther, Polyacetale, Thioplaste.

c) Polykondensationsprodukte oder Vorkondensate auf Basis von bi- oder polyfunktionellen Verbindungen mit kondensationsfähigen Gruppen, deren Homo- und Mischkondensationsprodukte sowie Produkte der Nachbehandlung, wofür beispielsweise genannt seien: Polyester, gesättigte (z. B. Polyäthylenterephthalat) oder ungesättigte (z. B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z. B. Alkydharze); Polyamide (z. B. Hexamethylendiamin-adipat), Maleinatharze, Melaminharze, Phenolharze, Anilinharze, Furanharze, Carbamidharze bzw. auch deren Vorkondensate und analog gebaute Produkte, Polycarbonate, Silikonharze und andere.

d) Polyadditionsprodukte wie Polyurethane (vernetzt und unvernetzt), Epoxydharze.

II. Halbsynthetische organische Materialien wie z. B. Celluloseester bzw. Mischester (Acetat, Propionat), Nitrocellulose, Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen, wie Wolle, Baumwolle, Seide, Bast, Jute, Hanf, Felle und Haare, Leder, Holzmassen in feiner Verteilung, Naturharze (wie Kolophonium, insbesondere Lackharze), ferner Kautschuk, Guttapercha, Balata, sowie deren Nachbehandlungs- und Modifizierungsprodukte (z. B. durch Härtung, Vernetzung oder Pfropfung), Abbauprodukte (z. B. durch Hydrolyse, Depolymerisation), durch Abwandlung reaktionsfähiger Gruppen erhältliche Produkte (z. B. durch Acylierung, Halogenierung, Vernetzung usw.).

Die in Betracht kommenden organischen Materialien können in den verschiedenartigsten Verarbeitungszuständen (Rohstoffe, Halbfabrikate oder Fertigfabrikate) und Aggregatzuständen vorliegen. Sie können einmal in Form der verschiedenartigsten geformten Gebilde vorliegen, d. h. also z. B. vorwiegend dreidimensional ausgedehnte Körper wie Blökke, Platten, Profile, Rohre, Spritzgußformlinge oder verschiedenartigste Werkstücke, Schnitzel oder Granulate, Schaumstoffe; vorwiegend zweidimensional ausgebildete Körper wie Filme, Folien, Lacke, Bänder, Überzüge, Imprägnierungen und Beschichtungen oder vorwiegend eindimensional ausgebildete Körper wie Fäden, Fasern, Flocken, Borsten, Drähte. Die besagten Materialien können andererseits auch in ungeformten Zuständen in den verschiedenartigsten homogenen und inhomogenen Verteilungsformen und Aggregatzuständen vorliegen, z. B. als Pulver, Lösungen, Emulsionen, Dispersionen, Latices (Beispiele: Lacklösungen, Polymerisat-Dispersionen, Sole, Gelee, Kitte, Pasten, Wachse, Kleb- und Spachtelmassen usw.).

Fasermaterialien können als endlose Fäden, Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papier, Pappen oder Papiermassen usw. vorliegen.

Den erfindungsgemäß anzuwendenden Verbindungen kommt auch Bedeutung für die Behandlung von textilen organischen Materialien, insbesondere textilen Geweben, zu. Sofern Fasern, welche als Stapelfasern oder endlose Fasern, in Form von Strangen, Geweben, Gewirken, Vliesen, beflockten Substraten oder Verbundstoffen vorliegen können, erfindungsgemäß weiß zu tönen sind, so geschieht dies mit Vorteil in wässerigem Medium, worin die betreffenden Verbindungen in feinverteilter Form (Suspension, gegebenenfalls Lösung) vorliegen. Gegebenenfalls können bei der Behandlung Dispergiermittel zugesetzt werden, wie z. B. Seifen, Polyglykoläther von Fettalkoholen, Fettaminen oder Alkylphenolen, Cellulosesulfitablauge oder Kondensationsprodukte von gegebenenfalls alkylierten Napthalinsulfonsäuren mit Formaldehyd. Als besonders zweckmäßig erweist es sich, in neutralem, schwach alkalischem oder saurem Bade zu arbeiten. Ebenso ist es vorteilhaft, wenn die Behandlung bei erhöhten Temperaturen von etwa 50 bis 100° C, beispielsweise bei Siedetemperatur des Bades oder in deren Nähe (etwa 90° C) erfolgt. Für die erfindungsgemäße Veredelung kommen auch Lösungen in organischen Lösungsmitteln in Betracht, wie dies in der Färbereipraxis in der sogenannten Lösungsmittelfärberei (Foulard-Thermofixierapplikation, Ausziehfärbeverfahren in Trommelfärbemaschinen) beispielsweise für Polyamid- und Polyester-Substrate praktiziert wird.

Die erfindungsgemäß zu verwendenden neuen Weißtöner können ferner den Materialien vor oder während deren Verformung zugesetzt bzw. einverleibt werden. So kann man sie beispielsweise bei der Herstellung von Filmen, Folien, Bändern oder Formkörpern der Pressmasse oder Spritzgußmasse beifügen oder vor dem Verspinnen in der Spinnmasse lösen, dispergieren oder anderweitig für eine homogene Feinverteilung sorgen. Die Weißtöner können auch den Ausgangssubstanzen, Reaktionsgemischen oder Zwischenprodukten zur Herstellung voll- oder halbsynthetischer organischer Materialien zugesetzt werden, also auch vor oder während der chemischen Umsetzung, beispielsweise bei einer Polykondensation (also auch Vorkondensaten), bei einer Polymerisation (also auch Prepolymeren) oder einer Polyaddition.

Die neuen Weißtöner können selbstverständlich auch überall dort eingesetzt werden, wo organische Materialien der oben angedeuteten Art mit anorganischen Materialien in irgendeiner Form kombiniert werden (typische Beispiele: Waschmittel, Weißpigmente in organischen Substanzen).

Die neuen weißtönenden Substanzen zeichnen sich durch besonders gute Hitzebeständigkeit, Lichtechtheit und Migrierbeständigkeit aus.

Die Menge der erfindungsgemäß zu verwendenden neuen Weißtöner, bezogen auf das weiß zu tönende Material, kann in weiten Grenzen schwanken. Schon mit sehr geringen Mengen, in gewissen Fällen z. B. solchen von 0,001 Gew.-%, kann ein deutlicher und haltbarer Effekt erzielt werden. Es können aber auch Mengen bis zu etwa 0,5 Gew.-% und mehr zur Anwendung gelangen. Für die meisten praktischen Belange sind vorzugsweise Mengen zwischen 0,01 und 0,2 Gew.-% von Interesse.

Die neuen als Weißtöner dienenden Verbindungen können beispielsweise auch wie folgt eingesetzt werden:

a) In Mischungen mit Farbstoffen oder Pigmenten oder als Zusatz zu Färbebädern, Druck-, Ätz- oder Reservepasten. Ferner auch zur Nachbehandlung von Färbungen, Drucken oder Ätzdrucken.

b) In Mischungen mit sogenannten „Carriern", Antioxydantien, Lichtschutzmitteln, Hitzestabilisatoren, chemischen Bleichmitteln oder als Zusatz zu Bleichbädern.

c) In Mischung mit Vernetzern, Appreturmitteln wie Stärke oder synthetisch zugänglichen Appreturen. Die erfindungsgemäßen Erzeugnisse können vorteilhaft auch den zur Erzielung einer knitterfesten Ausrüstung benützten Flotten zugesetzt werden.

d) In Kombination mit Waschmitteln. Die Waschmittel und Aufhellmittel können den zu benützenden Waschbädern getrennt zugefügt werden. Es ist auch vorteilhaft, Waschmittel zu verwenden, die die Weißtöner beigemischt enthalten. Als Waschmittel eignen sich beispielsweise Seifen, Salze von Sulfonatwaschmitteln, wie z. B. von sulfonierten an 2-Kohlenstoffatom durch höhere Alkylreste substituierten Benzimidazolen, ferner Salze von Monocarbonsäureestern der 4-Sulfophthalsäure mit höheren Fettalkoholen, weiterhin Salze von Fett-Alkoholsulfonaten, Alkylarylsulfonsäuren oder Kondensationsprodukten von höheren Fettsäuren mit aliphatischen Oxy- oder Aminosulfonsäuren. Ferner können nicht-ionogene Waschmittel herangezogen werden, z. B. Polyglykoläther, die sich von Äthylenoxid und höheren Fettalkoholen, Alkylphenolen oder Fettaminen ableiten.

e) In Kombination mit polymeren Trägermaterialien (Polymerisations-, Polykondensations- oder Polyadditionsprodukten), in welche die Weißtöner gegebenenfalls neben anderen Substanzen in gelöster oder dispergierter Form eingelagert sind, z. B. bei Beschichtungs-, Imprägnier- oder Bindemitteln (Lösungen, Dispersionen, Emulsionen), Textilien, Vliese, Papier, Leder.

f) Als Zusätze zu den verschiedensten industriellen Produkten, um dieselben marktfähiger zu machen oder Nachteile in der Gebrauchsfähigkeit zu vermeiden, z. B. als Zusatz zu Leimen, Klebemitteln, Zahnpasten, Anstrichstoffen usw.

g) In Kombination mit anderen, weißtönend wirkenden Substanzen (z. B. zwecks Nuancen-Veränderung).

h) In Spinnbadpräparationen, d. h. als Zusätze zu Spinnbädern wie sie zur Gleitfähigkeitsverbesserung für die Weiterverarbeitung von Synthese-Fasern verwendet werden.

Die Verbindungen der eingangs angegebenen Formel lassen sich als Scintillatoren, für verschiedene Zwecke photographischer Art, wie für die elektrophotographische Reproduktion oder zur Supersensibilisierung verwenden.

Wird das Weißtönverfahren mit anderen Behandlungs- oder Veredelungsmethoden kombiniert, so erfolgt die kombinierte Behandlung vorteilhaft mit Hilfe entsprechender beständiger Präparate. Solche Präparate sind dadurch gekennzeichnet, daß sie weißtönende Verbindungen der eingangs angegebenen allgemeinen Formel sowie Dispergiermittel, Waschmittel, Carrier, Farbstoffe, Pigmente oder Appreturmittel enthalten.

Bei Behandlung von einer Reihe von Fasersubstraten, z. B. von Polyesterfasern, mit den erfindungsgemäßen Weißtönern verfährt man zweckmäßig dergestalt, daß man diese Fasern mit den wässerigen Dispersionen der Weißtöner bei Temperaturen unter 75° C, z. B. bei Raumtemperatur, imprägniert und einer trockenen Wärmebehandlung bei Temperaturen über 100° C unterwirft, wobei es sich im allgemeinen empfiehlt, das Fasermaterial vorher noch bei mäßig erhöhter Temperatur, z. B. bei mindestens 60° C bis etwa 100° C, zu trocknen. Die Wärmebehandlung in trockenem Zustande erfolgt dann vorteilhaft bei Temperaturen zwischen 120 und 225° C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einen einzigen Arbeitsgang zusammengelegt werden.

Beispiel 1

Zu einer Lösung von 22,3 g (0,1 Mol) 2-(4-Formylphenyl)-benzoxazol und 31,2 g (0,1 Mol) 2,3-Dimethoxy-6-dimethoxy-phosphonomethylchinoxalin in 300 ml Dimethylformamid gibt man innerhalb von 20 Minuten portionsweise 12 g (0,22 Mol) Natriummethylat. Man läßt 4 Stunden bei 50° C rühren, trägt das Reaktionsgemisch dann auf 1 l Eiswasser aus und stellt mit Essigsäure auf pH 4 bis 5. Nach dem Abkühlen wird der gelbliche Niederschlag abgesaugt, mit Wasser und Methanol gewaschen und getrocknet. Man erhält so 28,5 g (70% der Theorie) an Rohpro-

dukt der Formel

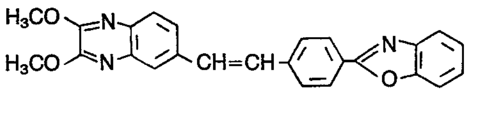
(1)

das durch mehrmaliges Umkristallisieren aus Xylol gereinigt wird. Die Substanz fluoresziert in Dimethylformamid gelöst rotstichig blau und besitzt, eingearbeitet in Polyäthylenterephthalat, einen starken aufhellenden Effekt mit guten Echtheiten.

Die verwendete Dimethoxyphosphonomethyl-Verbindung der Formel

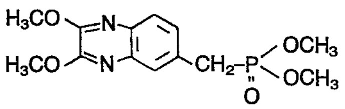
(2)

wird auf folgende Weise hergestellt:

Zu einer Suspension von 106,5 g (0,5 Mol) 2,3-Dichlor-6-methylchinoxalin in 1 l Methanol tropft man bei 20 bis 30° C innerhalb einer Stunde eine Lösung von 1 Mol Natriummethylat in 1 l Methanol. Man rührt 5 Stunden bei 40° C und destilliert das Lösungsmittel im Vakuum ab. Der Rückstand wird mit 1 l Wasser versetzt und nach dem Abkühlen abfiltriert. Man erhält 95,6 g (94% der Theorie) farblose Kristalle vom Schmp. 81° C, aus Methanol umkristallisiert 81 bis 82° C.

Zu einer Lösung von 40,8 g (0,2 Mol) 2,3-Dimethoxy-6-methyl-chinoxalin und 0,2 g Dibenzoylperoxid in 300 ml wasserfreiem Tetrachlorkohlenstoff gibt man bei 60° C portionsweise innerhalb von 20 Minuten eine Mischung aus 35,6 g (0,2 Mol) N-Bromsuccinimid und 0,2 g Azoisobutyronitril und rührt bei Rückflußtemperatur 4 Stunden. Danach wird das Succinimid abfiltriert, der Filterkuchen mit heißem Tetrachlorkohlenstoff nachgewaschen und das Filtrat bis fast zur Trockne eingedampft. Der Rückstand wird abfiltriert und mit Petroläther (40 bis 80° C) gewaschen. Man erhält 41,4 g (73% der Theorie) Brommethylverbindung vom Schmp. 149° C.

70,6 g (0,25 Mol) rohes 2,3-Dimethoxy-6-brommethylchinoxalin werden unter Rühren mit 185 g Trimethylphosphit unter einer Stickstoffatmosphäre langsam auf 140° C erwärmt und dann 4 Stunden bei dieser Temperatur gerührt. Anschließend wird der größte Teil des überschüssigen Trimethylphosphits im Vakuum abdestilliert und der Rückstand mit 100 ml Cyclohexan versetzt. Man kühlt auf 0° C und läßt das Produkt auskristallisieren. Man erhält 66,5 g (86% der Theorie) farblose Kristalle vom Schmp. 114° C, die aus Methylcyclohexan umkristallisiert bei 114 bis 115° C schmelzen.

Analog wird die Phosphono-Verbindung der Formel

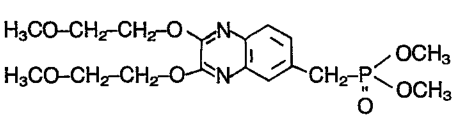
(3)

dargestellt.

Die Herstellung von 2-(4-Formylphenyl)-benzoxazol erfolgte in bekannter Weise durch Bromierung von 2-Tolylbenzoxazol und nachfolgender Umsetzung mit Hexamethylentetramin in Essigsäure.

Beispiel 2

Analog Beispiel 1 ergibt die Umsetzung von (3) mit 2-(4-Formylphenyl)-benzoxazol 31,3 g (63% der Theorie) der Verbindung der Formel

(4)
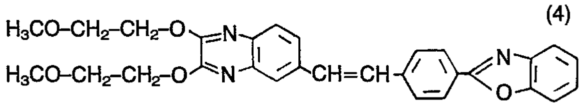

als gelbe Kristalle. Sie lassen sich durch Umlösen aus Toluol unter Zusatz von Bleicherde reinigen, Fluoreszenz in Dimethylformamid: rotstichig blau.

Beispiel 3

In gleicher Weise wie in Beispiel 1 erhält man aus 25,7 g (0,1 Mol) 5-Chlor-2-(4-formylphenyl)-benzoxazol und 31,2 g (0,1 Mol) 2,3-Dimethoxy-6-dimethoxyphosphonomethylchinoxalin, 33,9 g (76% der Theorie) der Verbindung der Formel

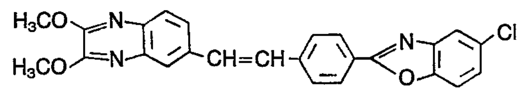
(5)

Aus Chlorbenzol erhält man blaßgelbe Kristalle, deren Lösung in Dimethylformamid eine intensive rotstichig blaue Fluoreszenz zeigt.

Beispiel 4

Zu einer Suspension von 38 g (0,2 Mol) 2,3-Dihydroxy-6-formylchinoxalin (hergestellt aus 2,3-Dichlor-6-brommethyl-chinoxalin und 5%iger Salpetersäure) und 63,5 g (0,2 Mol) 2-(4-Dimethoxyphosphonomethylphenyl)-benzoxazol in 500 ml Dimethylformamid gibt man innerhalb von 30 Minuten portionsweise 24 g (0,44 Mol) Natriummethylat. Man läßt 5 Stunden bei 50° C rühren, trägt das Reaktionsgemisch auf 1,5 l Eiswasser aus und stellt mit konzentrierter Salzsäure auf pH 2 bis 3. Der gelbliche Niederschlag wird abgesaugt, mit Wasser gewaschen und getrocknet. Man erhält 63 g (83% der Theorie) der Verbindung, die in einer der möglichen tautomeren Formen der Formel

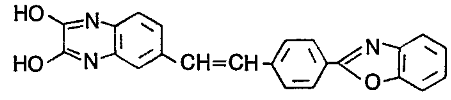
(6)

entspricht.

Die Verbindung wird durch Umkristallisieren aus Dimethylformamid gereinigt.

Das Rohprodukt der Formel (6) wird mit der fünffachen Menge an Thionylchlorid und katalytischen Mengen Dimethylformamid 4 Stunden unter Rückfluß gekocht, wonach das überschüssige Thionylchlorid im Vakuum abdestilliert wird. Der Rückstand wird mit Eiswasser gewaschen und abfiltriert. Man erhält 62,3 g (75% der Theorie) der Verbindung der Formel

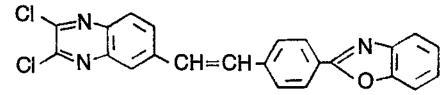
(7)

41,8 g (0,1 Mol) Rohprodukt der Formel (7) werden mit 10,5 g (0,1 Mol) Triäthylamin in 200 ml Dioxan suspendiert und bei Raumtemperatur tropfenweise

mit 8,6 g (0,1 Mol) Morpholin versetzt. Man rührt 2 Stunden bei 50° C, gibt anschließend auf 500 ml Wasser und filtriert ab. Man erhält 38,5 g (82% der Theorie) blaßgelbe Kristalle der Verbindung der Formel

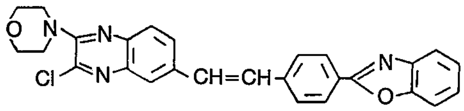
(8)

die durch Umlösen aus Chlorbenzol gereinigt wird.

Zu einer Lösung von 46,9 g (0,1 Mol) der Verbindung der Formel (8) in 200 ml Dimethylformamid gibt man tropfenweise 18 g 30%ige Natriummethylat-Lösung und rührt bei 30 bis 40° C 3 Stunden. Danach wird das Reaktionsgemisch auf 500 ml Wasser ausgetragen. Der Niederschlag wird abfiltriert, mit Wasser gewaschen und getrocknet. Man erhält 40 g (86% der Theorie) hellgelbes Kristallpulver der Verbindung der Formel

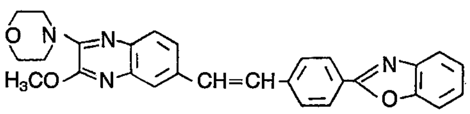
(9)

das aus Xylol umkristallisiert wird. In Dimethylformamid fluoresziert die Verbindung neutral blau.

Beispiel 5

In analoger Weise erhält man aus der Verbindung der Formel (7) durch Umsetzung mit a) Anilin und b) Natriumäthylat die Verbindung der Formel

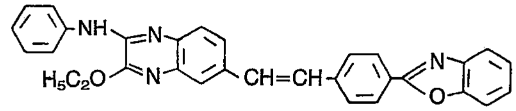
(10)

in Form gelber Kristalle, die aus Chlorbenzol umkristallisiert werden und in Dimethylformamid eine stark blaue Fluoreszenz zeigen.

In der nachstehenden Tabelle I werden weitere besonders wertvolle, erfindungsgemäß hergestellte Verbindungen, welche der Formel

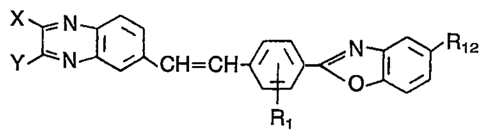
(11)

entsprechen, aufgeführt.

Tabelle I

| Verbindung | X | Y | $R_1$ | $R_{12}$ | Fluoreszenzfarbe in DMF |
|---|---|---|---|---|---|
| 12 | Äthoxy | Äthoxy | H | H | rotst. blau |
| 13 | Isopropoxy | Isopropoxy | 2-Chlor | H | rotst. blau |
| 14 | Butoxy | Butoxy | 2-Äthoxycarbonyl | H | rotviolett |
| 15 | $(OC_2H_4)_2$–$OCH_3$ | $(OC_2H_4)_2$–$OCH_3$ | H | tert.-Butyl | grünstichig blau |
| 16 | Methylamino | Methoxy | H | Methoxy | blau |
| 17 | N-Morpholino | Äthoxy | 2-Methoxy | H | blau |
| 18 | Diäthylamino | Isopropoxy | 2-Methoxycarbonyl | H | blau |
| 19 | N-Morpholino | N-Morpholino | H | Carboxy | tiefblau |
| 20 | Anilino | Chlor | H | Cyano | blau |
| 21 | Diäthylamino | Methoxy | 2-Cyano | H | blau |
| 22 | Anilino | Anilino | H | Methyl | grünst. blau |
| 23 | Piperidino | Butoxyäthoxy | 3-Chlor | Cyclohexyl | rotst. blau |
| 24 | Benzyloxy | Benzyloxy | H | Benzyl | blau |

Beispiel 6

31,1 g (0,11 Mol) 2,3-Dimethoxy-6-brommethylchinoxalin, 56 g Triäthylphosphit und 100 ml Dimethylformamid werden 4 Stunden auf 120 bis 150° C erhitzt. Danach destilliert man im Vakuum das überschüssige Triäthylphosphit und die Hauptmenge Dimethylformamid ab. Der Rückstand und 26,3 g (0,1 Mol) 2-(4-Formylphenyl)-4-methyl-5-phenyl-2H-1,2,3-triazol werden in 250 ml Dimethylformamid gelöst und tropfenweise mit 39,6 g 30%iger Natriummethylatlösung versetzt. Man rührt 3 Stunden bei 50° C, verdünnt mit 250 ml Methanol und stellt mit Essigsäure auf pH 7. Nach Abkühlen auf 0° C wird der hellgelbe Niederschlag abgesaugt, mit Methanol gewaschen, getrocknet und aus Xylol umkristallisiert. Man erhält 28,5 g (63% der Theorie) der Verbindung der Formel

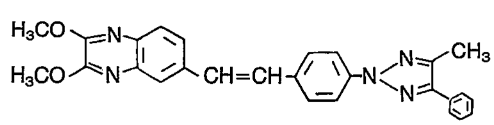
(25)

die in Dimethylformamid gelöst blau fluoresziert.

Beispiel 7

24,6 g (0,1 Mol) 2,3-Diäthoxy-6-formylchinoxalin und 37,1 g (0,1 Mol) 2-(4-Diäthoxyphosphonomethyl-phenyl)-4-phenyl-2H-1,2,3-triazol werden in 250 ml Dimethylformamid gerührt und bei Raumtemperatur mit 36 g 30%iger Natriummethylatlösung versetzt. Unter starker Violettfärbung erwärmt sich das Reaktionsgemisch auf 30° C. Man rührt 5 Stunden bei ca. 50° C, verdünnt hierauf mit 200 ml Methanol und stellt mit Essigsäure auf pH 7. Nach dem Abfiltrieren, Waschen mit Methanol und Trocknen erhält man 39,4 g (85% der Theorie) fahlgelbe Kristalle der Verbindung der Formel

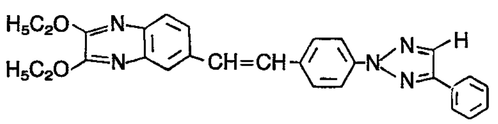

(26)

die aus Xylol-Bleicherde umkristallisiert, in Dimethylformamid gelöst eine stark blaue Fluoreszenz zeigt.

Der verwendete Aldehyd der Formel

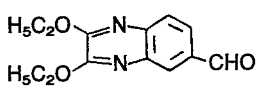

(27)

wird wie folgt synthetisiert:

31,1 g (0,1 Mol) rohes 2,3-Diäthoxy-6-brommethyl-chinoxalin werden mit 15,5 g (0,11 Mol) Hexamethylentetramin in 100 ml Chloroform 4 Stunden unter Rückfluß gekocht. Danach destilliert man 50 ml Chloroform ab, kühlt und fügt 50 ml Aceton hinzu. Nach Abfiltrieren bekommt man 40,2 g Urotropinsalz, das in 100 ml 50%iger Essigsäure 2 Stunden am Rückfluß erhitzt wird. Mit ca. 10 bis 20 ml konzentrierter Salzsäure wird die Lösung auf pH 3 gestellt, nach kurzem Aufkochen auf 0° C abgekühlt und mit 500 ml Wasser versetzt. Der ausgefallene Niederschlag wird abfiltriert, mit Wasser neutral gewaschen und getrocknet. Man erhält 16 g (73,3% der Theorie) farblose Kristalle, die nach dem Umkristallisieren aus Methylglykol bei 163° C schmilzen.

Analog wird auch der Aldehyd der Formel

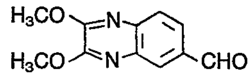

(28)

mit dem Schmelzpunkt 186° C in 65%iger Ausbeute hergestellt.

Beispiel 8

Analog Beispiel 7 erhält man aus dem Aldehyd (28) und dem entsprechenden Phosphonat die Verbindung der Formel

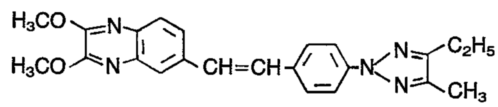

(29)

als gelbe Kristalle, die aus Xylol umkristallisiert werden und in Dimethylformamid grünstichig blau fluoreszieren.

Analog Beispielen 6 und 7 entstehen bei der Verwendung der entsprechenden Aldehyde die in der nachfolgenden Tabelle II aufgeführten Derivate der allgemeinen Formel

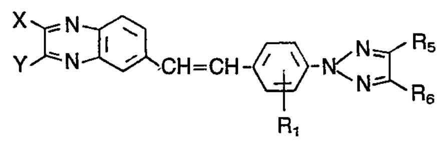

(30)

Tabelle II

| Verbindung | X | Y | $R_1$ | $R_5$ | $R_6$ | Fluoreszenz in DMF |
|---|---|---|---|---|---|---|
| 31 | Butoxy | Butoxy | H | Methyl | H | rotst. blau |
| 32 | Chlor | Dimethyl-amino | 2-Chlor | Methyl | Methyl | blau |
| 33 | Morpholino | Morpholino | H | Äthyl | Methyl | blauviolett |
| 34 | Anilino | Äthoxy | H | Methyl | Phenyl | rotst. blau |
| 35 | Piperidino | Methoxy | 2-Cyano | H | Phenyl | blau |
| 36 | Anilino | Anilino | H | Phenyl | Phenyl | violett |
| 37 | n-Propoxy | Äthylamino | 2-Carb-äthoxy | H | Styryl | rotviolett |
| 38 | 2-Hydroxy-äthylamino | Butoxy | H | Phenyl | Chlor | rotst. blau |
| 39 | Methyl-amino | Methoxy | H | Phenyl | Äthoxy-carbonyl | blau |
| 40 | Benzyl-amino | Benzyl-amino | H | Phenyl | Cyano | tiefblau |

Beispiel 9

Analog Beispiel 7 erhält man aus 2,3-Dimethoxy-6-formylchinoxalin und 5,6-Dimethoxy-2-(4-diäthoxyphosponomethylphenyl)-2H-benzotriazol die Verbindung der Formel

(41)

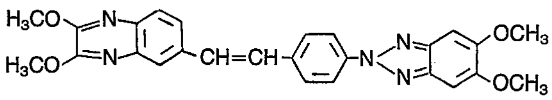

als schwach gelbe Kristalle, die in Dimethylformamid eine blaue Fluoreszenz zeigen.

Beispiel 10

In analoger Weise wird die Verbindung der Formel

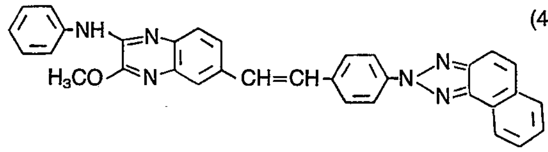

(42)

in Form hellgelber Kristalle gewonnen, die in Dimethylformamid gelöst rötlich blau fluoreszieren. Analog erhält man die in der Tabelle III aufgeführten 2H-Benzotriazolderivate der allgemeinen Formel

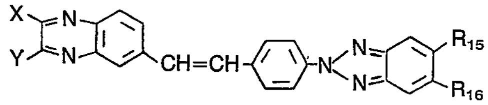

(43)

Tabelle III

| Verbindung | X | Y | $R_{15}$ | $R_{16}$ | Fluoreszenz in DMF |
|---|---|---|---|---|---|
| 44 | Diäthyl-amino | Chlor | Methyl | H | grünst. blau |
| 45 | Methoxy-äthoxy | Methoxy-äthoxy | Methoxy | H | blau |
| 46 | Diäthyl-amino | Äthoxy | Methoxy | Methoxy | blau |
| 47 | Piperidino | Piperidino | Brom | Methoxy | blau |
| 48 | Benzyloxy | Benzyloxy | Methyl | Methoxy | grünst. blau |
| 49 | Butoxy | Anilino | tert.-Butyl | H | grünst. blau |
| 50 | Methoxy | Methoxy | H | H | blauviolett |

Beispiel 11

31,2 g (0,11 Mol) 2,3-Dimethoxy-6-brommethylchinoxalin, 56 g Triäthylphosphit und 100 ml Dimethylformamid werden 4 Stunden bei 120 bis 150° C verrührt. Danach destilliert man im Vakuum das überschüssige Triäthylphosphit und die Hauptmenge des Dimethylformamids ab. Der Rückstand und 15 g (0,1 Mol) 4-Formylbenzoesäure werden in 200 ml Dimethylformamid suspendiert. Dazu tropft man innerhalb von 20 Minuten 36 g 30%ige Natriummethylatlösung und rührt die Reaktionsmischung 3 Stunden bei 50° C. Danach wird abgekühlt, auf 500 ml Wasser ausgetragen und mit konzentrierter Salzsäure auf pH 2 bis 3 gestellt. Nach dem Abfiltrieren, Waschen mit Wasser und Trocknen erhält man 28,5 g (85% der Theorie) hellgelbe Kristalle der Formel

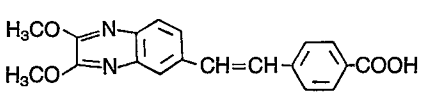

(51)

die durch Umkristallisieren aus Dimethylformamid gereinigt werden.

33,6 g (0,1 Mol) Rohprodukt der vorstehend beschriebenen Verbindung (51) werden mit 30 g Thionylchlorid und 2 g Dimethylformamid in 150 ml Xylol in das Säurechlorid überführt. Dann werden portionsweise 15 g (0,11 Mol) Benzoesäurehydrazid eingetragen. Nach dem Abklingen der Reaktion bringt man die Mischung langsam auf Rückflußtemperatur und kocht 3 Stunden. Dann wird abgekühlt und mit Wasserdampf destilliert. Man erhält so 35,2 g (81% der Theorie) der Verbindung der Formel

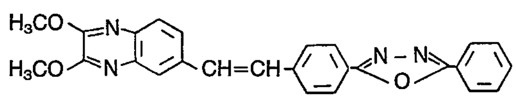

(52)

die in Dimethylformamid rotviolett fluoresziert.

Beispiel 12

Gemäß Beispiel 1 erhält man aus 5-Biphenylyl-2-(4-formylphenyl)-1,3,4-oxdiazol und 2,3-Dibutoxy-6-diäthoxyphosphonomethylchinoxalin die Verbindung der Formel

(53)

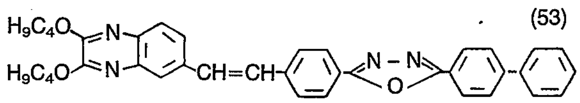

in 80%iger Ausbeute in Form hellgelber Kristalle, die aus Xylol umkristallisiert werden und in Dimethylformamid rotstichig blau fluoreszieren.

Ebenso werden die in der folgenden Tabelle IV aufgeführten Oxdiazolderivate der allgemeinen Formel

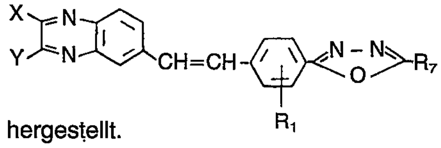

(54)

hergestellt.

Tabelle IV

| Verbindung | X | Y | $R_1$ | $R_7$ | Fluoreszenz in DMF |
|---|---|---|---|---|---|
| 55 | Äthoxy | Äthoxy | Cyano | Phenyl | blau |
| 56 | Anilino | Anilino | H | 4-Chlor-phenyl | blauviolett |
| 57 | N-Morpho-lino | Methoxy | H | 4-Äthoxy-carbonyl-phenyl | rotst. blau |
| 58 | Methyl-amino | Methoxy | Äthoxy-carbonyl | 4-Bipheny-lyl | blau |
| 59 | Benzyl-amino | Äthoxy | H | Styryl | rotviolett |
| 60 | Äthoxy | Dimethyl-amino | H | 4-Carboxy-phenyl | rotst. blau |
| 61 | Methoxy-äthoxy | Methoxy-äthoxy | H | 2,4-Dichlor-phenyl | rotst. blau |
| 62 | Butoxy | Äthylamino | H | 4-Methoxy-phenyl | rotst. blau |
| 63 | Benzyloxy | Benzyloxy | H | 4-tert.-Butylphenyl | |

Beispiel 13

Zu einer Lösung von 31,2 g (0,1 Mol) 2,3-Dimethoxy-6-dimethoxyphosphonomethylchinoxalin und 18,2 g (0,1 Mol) 4-Formylbiphenyl in 200 ml Dimethylformamid tropft man innerhalb von 20 Minuten 36 g 30%ige Natriummethylatlösung und läßt 5 Stunden bei 50° C rühren. Dann verdünnt man mit 100 ml Methanol und stellt mit Essigsäure auf pH 4 bis 5. Nach dem Abkühlen auf 0° C, Abfiltrieren und Trocknen erhält man 25,8 g (70% der Theorie) Rohprodukt der Formel

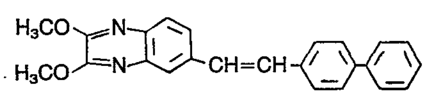

(64)

das durch Umkristallisieren aus Xylol mit Hilfe von Bleicherde gereinigt wird und in Dimethylformamid gelöst rotstichig blau fluoresziert.

Beispiel 14

Analog Beispiel 11 erhält man aus 2,3-Diäthoxy-6-formyl-chinoxalin und 2-Methoxy-4-formylbenzoesäure die Verbindung der Formel

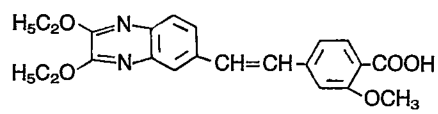

(65)

als schwach gelbe Kristalle, die in Dimethylformamid eine rotstichig blaue Fluoreszenz zeigen.

Ebenso lassen sich die in der Tabelle V aufgeführten Verbindungen der allgemeinen Formel

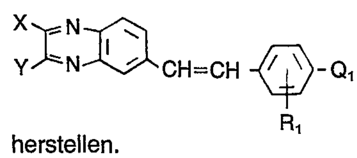

(66)

herstellen.

Tabelle V

| Verbindung | X | Y | $R_1$ | $Q_1$ | Fluoreszenz in DMF |
|---|---|---|---|---|---|
| 67 | Methoxy | Methoxy | 3-Cyano | Cyano | blau |
| 68 | Methoxy | Morpholino | 2-Cyano | Carbäthoxy | rotviolett |
| 69 | Dimethyl-amino | Äthoxy | 2-Chlor | Carbmethoxy | blau |
| 70 | 2-Hydroxy-äthylamino | 2-Hydroxy-äthylamino | 2-Methoxy | Cyano | rotst. blau |
| 71 | Methoxy-äthoxy-äthoxy | Methoxy-äthoxy-äthoxy | 3-Chlor | Anilino-carbonyl | blau |
| 72 | Butoxy | Butoxy | H | 4-Methoxy-styryl | rotviolett |

**Patentansprüche**

1. Fluoreszenz-Farbstoffe der Formel

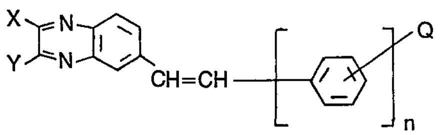

worin

X und Y Wasserstoff, Halogen, Alkyl, Aralkyl, Alkenyl, Hydroxy, Amino, Alkoxy, Aralkoxy, Cycloalkoxy, Aryloxy, Alkylmercapto, Alkylamino, Dialkylamino, Morpholino, Piperidino, Piperazino, Pyrrolidino, Acylamino oder Arylamino,

Q Wasserstoff, Pyrazol-1-yl, Oxazol-2-yl, Benzoxazol-2-yl, Naphthoxazol-2-yl, 1,2,4-Oxadiazol-5-yl, 1,3,4-Oxadiazol-2-yl, Isoxazol-3-yl, Isoxazol-5-yl, Thiazol-2-yl, Benzthiazol-2-yl, 1,3,4-Thiadiazol-2-yl, Imidazol-2-yl, Benzimidazol-2-yl, 1,2,3-Triazol-2-yl, 1,2,3-Triazol-4-yl, 1,2,4-Triazol-3-yl, 1,2,4-Triazol-5-yl, 1,3,5-Triazin-2-yl, 2H-Benzotriazol-2-yl, 2H-Naphthotriazol-2-yl, 1,2,3,4-Tetrazol-5-yl, 1,2,3,4-Tetrazol-1-yl, Benzo[b]-furan-2-yl, Naphtho[2,1-b]-furan-2-yl, Benzo[b]-thiophen-2-yl, Naphtho[2,1,-b]-thiophen-2-yl, Pyrimidin-2-yl, Pyridin-2-yl, Chinazolin-4-yl oder Chinazolin-2-yl, und

n 1 oder 2 bedeuten, wobei die Substituenten X, Y, Q und die übrigen cyclischen Reste durch für Weißtöner übliche nicht-chromophore Substituenten weiter substituiert sein können.

2. Fluoreszenz-Farbstoffe nach Anspruch 1, worin n 1 bedeutet.

3. Fluoreszenz-Farbstoffe nach Anspruch 1 der Formel

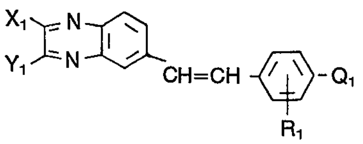

worin

$X_1$ und $Y_1$ Wasserstoff, Chlor, $C_1$–$C_4$-Alkylamino, Di-$C_1$–$C_4$-Alkylamino, Morpholino, Piperidino, gegebenenfalls durch Methyl, Methoxy oder Chlor substituiertes Phenylamino oder einen Rest der Formel

$$-(OCH_2-CH_2)_q-OR_2$$

$R_1$ Wasserstoff, Chlor, $C_1$–$C_4$-Alkyl, $C_1$–$C_4$-Alkoxy, $C_1$–$C_4$-Alkoxycarbonyl oder Cyano,

$R_2$ Wasserstoff, $C_1$–$C_4$-Alkyl, Benzyl oder Phenyl,

q eine ganze Zahl von 0 bis 7 und

$Q_1$ Chlor, Brom, Cyano, Carboxy, $C_1$–$C_4$-Alkoxycarbonyl oder einen Rest der Formel

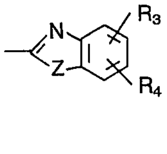 , 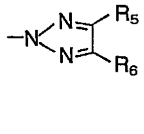 ,

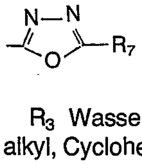 , 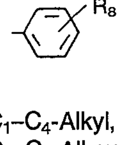

$R_3$ Wasserstoff, Chlor, $C_1$–$C_4$-Alkyl, Phenyl-$C_1$–$C_3$-alkyl, Cyclohexyl, Phenyl, $C_1$–$C_4$-Alkoxy, $C_1$–$C_4$-Alkylsulfonyl, $C_1$–$C_4$-Alkoxycarbonyl, Cyan oder Carboxy oder zusammen mit $R_4$ einen gegebenenfalls durch 1 bis 4 Methylgruppen substituierten ankondensierten 1-Cyclopenteno-, 1-Cyclohexeno- oder Benzoring,

$R_4$ Wasserstoff, Chlor oder Methyl oder zusammen mit $R_3$ einen gegebenenfalls durch 1 bis 4 Methylgruppen substituierten ankondensierten 1-Cyclopenteno-, 1-Cyclohexeno- oder Benzoring,

$R_5$ $C_1$–$C_4$-Alkyl, Phenyl oder Styryl oder zusammen mit $R_6$ einen gegebenenfalls durch $C_1$–$C_4$-Alkyl, $C_1$–$C_4$-Alkoxy oder Chlor substituierten ankondensierten Benzoring oder ankondensiertes Naphtho,

$R_6$ Wasserstoff, $C_1$–$C_4$-Alkyl oder Phenyl oder zusammen mit $R_5$ einen gegebenenfalls durch $C_1$–$C_4$-Alkyl, $C_1$–$C_4$-Alkoxy oder Chlor substituierten ankondensierten Benzoring oder ankondensiertes Naphtho,

$R_7$ gegebenenfalls durch $C_1$–$C_4$-Alkyl, $C_1$–$C_4$-Alkoxy, $C_1$–$C_4$-Alkoxycarbonyl, Cyan oder Chlor substituiertes Phenyl, Styrol, Biphenylyl oder Naphthyl,

$R_8$ Wasserstoff, $C_1$–$C_4$-Alkyl, $C_1$–$C_4$-Alkoxy, $C_1$–$C_4$-Alkoxycarbonyl, Cyan oder einen gegebenenfalls durch $C_1$–$C_4$-Alkoxycarbonyl, Cyan oder Chlor substituierten Benzoxazol-2-yl-Rest,

Z O, S oder $NR_9$ und

$R_9$ Wasserstoff, $C_1$–$C_4$-Alkyl, Acetyl, Benzoyl, Benzyl oder Phenyl bedeuten.

4. Fluoreszenz-Farbstoffe nach Anspruch 3 der Formel

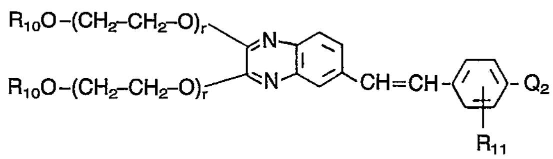

worin

$R_{10}$ $C_1$–$C_4$-Alkyl,

$R_{11}$ Wasserstoff oder Cyan,

r eine ganze Zahl von 0 bis 2,

$Q_2$ einen Rest der Formeln

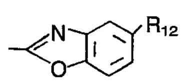 , 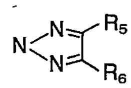 , 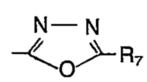

$R_{12}$ Wasserstoff, Chlor, $C_1$–$C_4$-Alkyl, Phenyl-$C_1$–$C_3$-alkyl, Cyclohexyl, Phenyl, $C_1$–$C_4$-Alkoxy, $C_1$–$C_4$-Alkylsulfonyl, $C_1$–$C_4$-Alkoxycarbonyl, Cyan oder Carboxy bedeuten und

$R_5$, $R_6$ und $R_7$ die in Anspruch 3 genannte Bedeutung haben.

5. Verfahren zur Herstellung von Fluoreszenzfarbstoffen gemäß Anspruch 1, dadurch gekennzeichnet, daß man entweder eine Phosphono-Verbindung der Formel

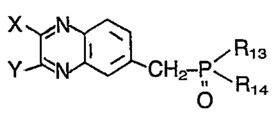

worin

X und Y die in Anspruch 1 genannte Bedeutung haben, der Benzolring weitere nicht-chromophore Substituenten aufweisen kann, und

$R_{13}$ und $R_{14}$ $C_1$–$C_4$-Alkoxy, $C_5$–$C_6$-Cycloalkoxy,

Phenyl oder Phenoxy bedeuten, mit einem Aldehyd der Formel

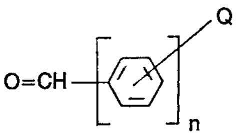

worin

Y und n die in Anspruch 1 genannte Bedeutung besitzen und der Benzolring weitere nicht-chromophore Substituenten aufweisen kann, oder eine Phosphono-Verbindung der Formel

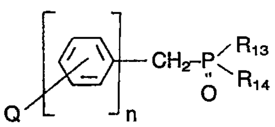

worin

Q, $R_{13}$, $R_{14}$ und n die in Anspruch 1 genannte Bedeutung besitzen und der Benzolring durch nicht-chromophore Substituenten substituiert sein kann, mit einem Aldehyd der Formel

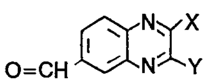

worin

X und Y die in Anspruch 1 genannte Bedeutung haben und der Benzolring durch nicht-chromophore Substituenten substituiert sein kann, in organischen Lösungsmitteln in Gegenwart basischer Kondensationsmittel, kondensiert.

6. Verfahren zum Weißtönen von synthetischen, halbsynthetischen und natürlichen organischen hochmolekularen Materialien, dadurch gekennzeichnet, daß man die Fluoreszenzfarbstoffe gemäß Anspruch 1 einsetzt.

7. Mit den Fluoreszenzfarbstoffen gemäß Anspruch 1 weißgetönte synthetische, halbsynthetische und natürliche organische hochmolekulare Materialien.

**Claims**

1. Fluorescent dyestuffs of the formula

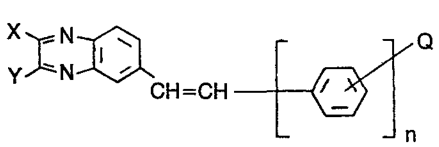

wherein

X and Y denote hydrogen, halogen, alkyl, aralkyl, alkenyl, hydroxyl, amino, alkoxy, aralkoxy, cycloalkoxy, aryloxy, alkylmercapto, alkylamino, dialkylamino, morpholino, piperidino, piperazino, pyrrolidino, acylamino or arylamino,

Q denotes hydrogen, pyrazol-1-yl, oxazol-2-yl, benzoxazol-2-yl, naphthoxazol-2-yl, 1,2,4-oxadiazol-5-yl, 1,3,4-oxadiazol-2-yl, isoxazol-3-yl, isoxazol-5-yl, thiazol-2-yl, benzthiazol-2-yl, 1,3,4-thiadiazol-2-yl, imidazol-2-yl, benzimidazol-2-yl, 1,2,3-triazol-2-yl, 1,2,3-triazol-4-yl, 1,2,4-triazol-3-yl, 1,2,4-triazol-5-yl, 1,3,5-triazin-2-yl, 2H-benzotriazol-2-yl, 2H-naphthotriazol-2-yl, 1,2,3,4-tetrazol-5-yl, 1,2,3,4-tetrazol-1-yl, benzo[b]-furan-2-yl, naphtho[2,1-b]-furan-2-yl, benzo[b]-thiophen-2-yl, naphtho[2,1-b]-thiophen-2-yl, pyrimidin-2-yl, pyridin-2-yl, quinazolin-4-yl or quinazolin-2-yl, and

n denotes 1 or 2,

it being possible for the substituents X, Y and Q and the remaining cyclic radicals to be further substituted by nonchromophoric substituents which are customary for whiteners.

2. Fluorescent dyestuffs according to Claim 1, wherein n denotes 1.

3. Fluorescent dyestuffs according to Claim 1, of the formula

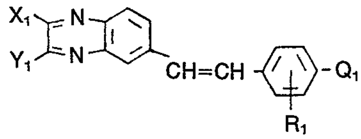

wherein

$X_1$ and $Y_1$ denote hydrogen, chlorine, $C_1$–$C_4$-alkylamino, di-$C_1$–$C_4$-alkylamino, morpholino, piperidino, phenylamino which is optionally substituted by methyl, methoxy or chlorine or a radical of the formula

$$-(OCH_2-CH_2)_q-OR_2$$

$R_1$ denotes hydrogen, chlorine, $C_1$–$C_4$-alkyl, $C_1$–$C_4$-alkoxy, $C_1$–$C_4$-alkoxycarbonyl or cyano,

$R_2$ denotes hydrogen, $C_1$–$C_4$-alkyl, benzyl or phenyl,

q denotes an integer from 0 to 7 and

$Q_1$ denotes chlorine, bromine, cyano, carboxyl, $C_1$–$C_4$-alkoxycarbonyl or a radical of the formula

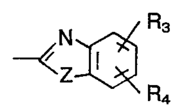 , 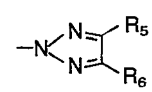 ,

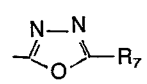 or 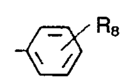

$R_3$ denotes hydrogen, chlorine, $C_1$–$C_4$-alkyl, phenyl-$C_1$–$C_3$-alkyl, cyclohexyl, phenyl, $C_1$–$C_4$-alkoxy, $C_1$–$C_4$-alkylsulphonyl, $C_1$–$C_4$-alkoxycarbonyl, cyano or carboxyl or, together with $R_4$, a fused-on 1-cyclopenteno, 1-cyclohexeno or benzo ring which is optionally substituted by 1 to 4 methyl groups,

$R_4$ denotes hydrogen, chlorine or methyl or, together with $R_3$, a fused-on 1-cyclopenteno, 1-cyclohexeno or benzo ring which is optionally substituted by 1 to 4 methyl groups,

$R_5$ denotes $C_1$–$C_4$-alkyl, phenyl or styryl or, together with $R_6$, a fused-on benzo ring which is optionally substituted by $C_1$–$C_4$-alkoxy or chlorine or fused-on naphtho,

$R_6$ denotes hydrogen, $C_1$–$C_4$-alkyl or phenyl or, together with $R_5$, a fused-on benzo ring which is optionally substituted by $C_1$–$C_4$-alkyl, $C_1$–$C_4$-alkoxy or chlorine or fused-on naphtho,

$R_7$ denotes phenyl, styryl, biphenylyl or naphthyl, optionally substituted by $C_1$–$C_4$-alkyl $C_1$–$C_4$-alkoxy, $C_1$–$C_4$-alkoxycarbonyl, cyano or chlorine,

$R_8$ denotes hydrogen, $C_1$–$C_4$-alkyl, $C_1$–$C_4$-alkoxy, $C_1$–$C_4$-alkoxycarbonyl, cyano or a benzoxazol-2-yl radical which is optionally substituted by $C_1$–$C_4$-alkoxycarbonyl, cyano or chlorine,

Z denotes O, S or $NR_9$ and

$R_9$ denotes hydrogen, $C_1$–$C_4$-alkyl, acetyl, benzoyl, benzyl or phenyl.

4. Fluorescent dyestuffs according to Claim 3, of the formula

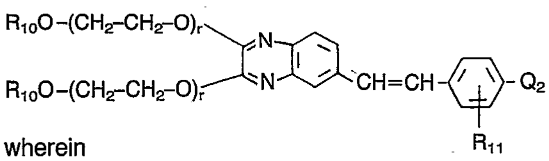

wherein

$R_{10}$ denotes $C_1$–$C_4$-alkyl,

$R_{11}$ denotes hydrogen or cyano,

r denotes an integer from 0 to 2,

$Q_2$ denotes a radical of the formulae

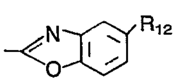 , 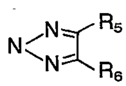 or 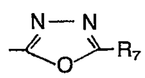

$R_{12}$ denotes hydrogen, chlorine, $C_1$–$C_4$-alkyl, phenyl-$C_1$–$C_3$-alkyl, cyclohexyl, phenyl, $C_1$–$C_4$-alkoxy, $C_1$–$C_4$-alkylsulphonyl, $C_1$–$C_4$-alkoxycarbonyl, cyano or carboxyl and

$R_5$, $R_6$ and $R_7$ have the meaning given in Claim 3.

5. Process for the preparation of fluorescent dyestuffs according to Claim 1, characterised in that either a phosphono compound of the formula

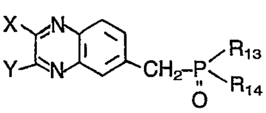

wherein

X and Y have the meaning given in Claim 1 and the benzene ring can contain further non-chromophoric substituents and

$R_{13}$ and $R_{14}$ denote $C_1$–$C_4$-alkoxy, $C_5$–$C_6$-cycloalkoxy, phenyl or phenoxy,

is subjected to a condensation reaction with an aldehyde of the formula

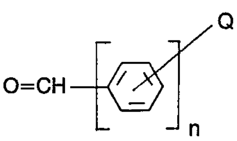

wherein

Y and n have the meaning given in Claim 1 and the benzene ring can contain further non-chromophoric substituents, or a phosphono compound of the formula

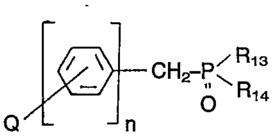

wherein

Q, $R_{13}$, $R_{14}$ and n have the meaning given in Claim 1 and the benzene ring can be substituted by non-chromophoric substituents, is subjected to a condensation reaction with an aldehyde of the formula

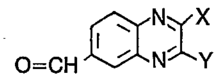

wherein

X and Y have the meaning given in Claim 1 and the benzene ring can be substituted by non-chromophoric substituents, in organic solvents in the presence of basic condensing agents.

6. Process for whitening synthetic, semi-synthetic and natural organic high-molecular materials, characterised in that the fluorescent dyestuffs according to Claim 1 are employed.

7. Synthetic, semi-synthetic and natural organic high-molecular materials whitened with the fluorescent dyestuffs according to Claim 1.

**Revendications**

1. Colorants fluorescents, caractérisés en ce qu'ils répondent à la formule:

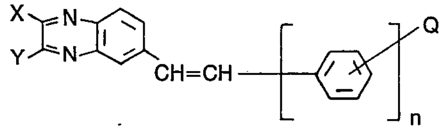

dans laquelle

X et Y représentent de l'hydrogène, un halogène, un groupe alkyle, aralkyle, alcényle, hydroxy, amino, alkoxy, aralkoxy, cycloalkoxy, aryloxy, alkylmercapto, alkylamino, dialkylamino, morpholino, pipéridino, pipérazino, pyrrolidino, acylamino, arylamino,

Q est un atome d'hydrogène ou un groupe pyrazole-1-yle, oxazole-2-yle, benzoxazole-2-yle, naphtoxazole-2-yle, 1,2,4-oxadiazole-5-yle, 1,3,4-oxadiazole-2-yle, isoxazole-3-yle, isoxazole-5-yle, thiazole-2-yle, benzothiazole-2-yle, 1,3,4-thiadiazole-2-yle, imidazole-2-yle, benzimidazole-2-yle, 1,2,3-triazole-2-yle, 1,2,3-triazole-4-yle, 1,2,4-triazole-3-yle, 1,2,4-triazole-5-yle, 1,3,5-triazine-2-yle, 2H-benzotriazole-2-yle, 2H-naphtotriazole-2-yle, 1,2,3,4-tétrazole-5-yle, 1,2,3,4-tétrazole-1-yle, benzo[b]-furanne-2-yle, naphto[2,1]-furanne-2-yle, benzo[b]-thiophène-2-yle, naphto[2,1-b]-thiophène-2-yle, pyrimidine-2-yle, pyridine-2-yle, quinazoline-4-yle ou quinazoline-2-yle, et

n est égal à 1 ou 2, les substituants X, Y, Q et les autres restes cycliques pouvant encore porter d'autres substituants non chromophores classiques pour des azurants optiques.

2. Colorants fluorescents suivant la revendication 1, caractérisés en ce que n est égal à 1.

3. Colorants fluorescents suivant la revendication 1, caractérisés en ce qu'ils répondent à la formule:

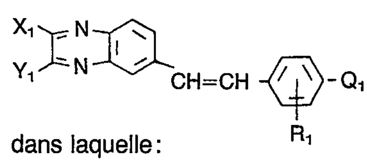

dans laquelle:

$X_1$ et $Y_1$ représentent de l'hydrogène, du chlore, un groupe alkylamino en $C_1$ à $C_4$, di-(alkyle en $C_1$ à $C_4$)-amino, morpholino, pipéridino, phénylamino éventuellement substitué par un radical méthyle, méthoxy ou chloro, ou un reste de formule:

$$-(OCH_2-CH_2)_q-OR_2$$

$R_1$ est un atome d'hydrogène ou de chlore ou un groupe alkyle en $C_1$ à $C_4$, alkoxy en $C_1$ à $C_4$, (alkoxy en $C_1$ à $C_4$)-carbonyle ou cyano,

$R_2$ est un atome d'hydrogène ou un groupe alkyle en $C_1$ à $C_4$, benzyle ou phényle,

q est un nombre entier ayant une valeur de 0 à 7 et

$Q_1$ est un radical chloro, bromo, cyano, carboxy, (alkoxy en $C_1$ à $C_4$)-carbonyle ou un reste de formule:

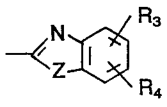 , 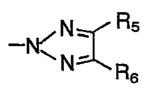 ,

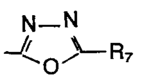 ou 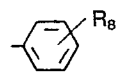

$R_3$ représente de l'hydrogène, du chlore, un groupe alkyle en $C_1$ à $C_4$, phényl-(alkyle en $C_1$ à $C_3$), cyclohexyle, phényle, alkoxy en $C_1$ à $C_4$, (alkyle en $C_1$ à $C_4$)-sulfonyle, (alkoxy en $C_1$ à $C_4$)-carbonyle, cyano ou carboxy ou forme avec $R_4$ un noyau 1-cyclopenténique, 1-cyclohexénique ou benzénique condensé, substitué le cas échéant par 1 à 4 groupes méthyle,

$R_4$ représente de l'hydrogène, du chlore ou un groupe méthyle ou forme avec $R_3$ un noyau 1-cyclopenténique, 1-cyclohexénique ou benzénique condensé, éventuellement substitué par 1 à 4 groupes méthyle,

$R_5$ est un groupe alkyle en $C_1$ à $C_4$, phényle ou styryle ou forme avec $R_6$ un noyau benzénique condensé éventuellement substitué par un groupe alkyle en $C_1$ à $C_4$, un groupe alkoxy en $C_1$ à $C_4$ ou du chlore, ou un noyau naphtalénique condensé,

$R_6$ représente de l'hydrogène, un groupe alkyle en $C_1$ à $C_4$ ou un groupe phényle ou forme avec $R_5$ un noyau benzénique condensé, éventuellement substitué par un groupe alkyle en $C_1$ à $C_4$, un groupe alkyle en $C_1$ à $C_4$, un groupe alkoxy en $C_1$ à $C_4$ ou du chlore, ou un noyau naphtalénique condensé,

$R_7$ est un groupe phényle éventuellement substitué par un radical alkyle en $C_1$ à $C_4$, alkoxy en $C_1$ à $C_4$, (alkoxy en $C_1$ à $C_4$)-carbonyle, cyano ou chloro ou un groupe styryle, biphénylyle ou naphtyle,

$R_8$ est un atome d'hydrogène, un groupe alkyle en $C_1$ à $C_4$, alkoxy en $C_1$ à $C_4$, (alkoxy en $C_1$ à $C_4$)-carbonyle, cyano, ou un reste benzoxazole-2-yle éventuellement substitué par un radical (alkoxy en $C_1$ à $C_4$)-carbonyle, cyano ou chloro,

Z représente un atome d'oxygène ou de soufre ou un groupe $NR_9$ et

$R_9$ est un atome d'hydrogène ou un groupe alkyle en $C_1$ à $C_4$, acétyle, benzoyle, benzyle ou phényle.

4. Colorants fluorescents suivant la revendication 3, caractérisés en ce qu'ils répondent à la formule:

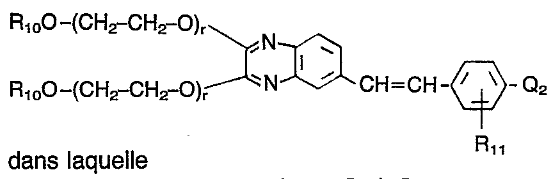

dans laquelle

$R_{10}$ est un groupe alkyle en $C_1$ à $C_4$,

$R_{11}$ est un atome d'hydrogène ou un groupe cyano,

r est un nombre entier égal à 0, 1 ou 2,

$Q_2$ est un reste de formule:

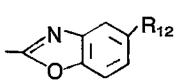 , 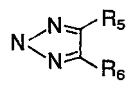 ou 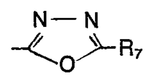

$R_{12}$ représente de l'hydrogène, du chlore, un groupe alkyle en $C_1$ à $C_4$, phényl-(alkyle en $C_1$ à $C_3$), cyclohexyle, phényle, alkoxy en $C_1$ à $C_4$, (alkyle en $C_1$ à $C_4$)-sulfonyle, (alkoxy en $C_1$ à $C_4$)-carbonyle, cyano ou carboxy et

$R_5$, $R_6$ et $R_7$ ont les définitions données dans la revendication 3.

5. Procédé de production de colorants fluorescents suivant la revendication 1, caractérisé en ce qu'il consiste à condenser ou bien un composé phosphono de formule:

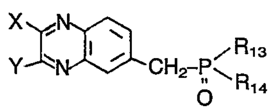

(dans laquelle

X et Y ont les définitions données dans la revendication 1, le noyau benzénique pouvant porter d'autres substituants non chromophores, et

$R_{13}$ et $R_{14}$ représentent un groupe alkoxy en $C_1$ à $C_4$, cycloalkoxy en $C_5$ ou $C_6$, phényle ou phénoxy), avec un aldéhyde de formule:

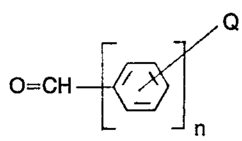

(dans laquelle

Y et n ont les définitions données dans la revendication 1 et le noyau benzénique peut porter d'autres substituants non chromophores), ou bien un composé phosphono de formule:

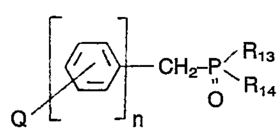

(dans laquelle

Q, $R_{13}$ et $R_{14}$ et n ont les définitions données dans la revendication 1 et le noyau benzénique peut porter

d'autres substituants non chromophores) avec un aldéhyde de formule:

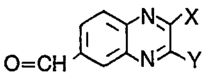

(dans laquelle

X et Y ont les définitions données dans la revendication 1 et le noyau benzénique peut porter des substituants non chromophores), dans des solvants organiques, en présence d'agents basiques de condensation.

6. Procédé d'azurage optique de matières organiques de haut poids moléculaire, synthétiques, semi-synthétiques et naturelles, caractérisé en ce qu'il consiste à utiliser les colorants fluorescents suivant la revendication 1.

7. Des matières organiques de haut poids moléculaire synthétiques, semi-synthétiques et naturelles azurées optiquement avec les colorants fluorescents suivant la revendication 1.